# FASCICULE DE BREVET EUROPEEN

(11) **EP 1 194 096 B1**
(45) Date de publication et mention de la délivrance du brevet: **03.12.2003**
(21) Numéro de dépôt: 00931500.3
(22) Date de dépôt: 13.06.2000
(51) Int. Cl.: A61F 13/02

(54) **PANSEMENT ADHESIF**
SELBSTKLEBENDER WUNDVERBAND
ADHESIVE BANDAGE

(30) Priorité: 16.06.1999 FR 9907800
(43) Date de publication de la demande: 10.04.2002
(73) Titulaire: Internova International Innovation Company B.V., 3012 CA Rotterdam (NL)
(72) Inventeur: LEMAIRE, Serge, F-34980 Saint-Clément-de-Rivière (FR)
(74) Mandataire: Nithardt, Roland
(86) Numéro de dépôt international: IB0000797
(87) Numéro de publication internationale: WO00076440

(56) Documents cités:
- EP-A- 0 308 122
- WO-A-97/28771
- GB-A- 2 224 445
- US-A- 5 397 297

## Description

### Domaine technique

La présente invention concerne un pansement adhésif, en particulier pour une application sur une zone affectée critique de l'épiderme, très sensible à une contamination par des germes microbiens et devant rester stérile, notamment une zone comportant un site d'implantation d'une fistule artérioveineuse formant un abord vasculaire d'un patient en hémodialyse, ledit pansement adhésif comprenant un support ayant une face adhésive, un tampon stérile disposé sur ladite face adhésive du support et un film de protection recouvrant la face adhésive et le tampon stérile, ledit film de protection étant formé d'une première et d'une deuxième languette détachable, ladite première languette détachable étant repliée sur elle-même selon une ligne de pliage, en dehors de la zone du tampon stérile, et comportant un premier volet appliqué sur la face adhésive du support et qui s'étend au moins d'une première extrémité dudit support vers l'intérieur de celui-ci jusqu'à ladite ligne de pliage de manière à recouvrir entièrement le tampon stérile, et un second volet constituant une amorce d'arrachage dudit premier volet et qui s'étend sur ledit premier volet depuis ladite ligne de pliage en direction de ladite première extrémité du support, ladite deuxième languette détachable comportant un troisième volet appliqué sur la face adhésive du support et qui s'étend, en dehors de la zone du tampon stérile, au moins d'une seconde extrémité dudit support, opposée à ladite première extrémité, vers l'intérieur dudit support jusqu'à ladite ligne de pliage de la première languette détachable.

### Technique antérieure

Un pansement adhésif du type tel que décrit ci-dessus est présenté par le brevet américain US-A-5 397 297. Sa deuxième languette détachable, plus courte que la première, est aussi repliée sur elle-même en forme de V. Elle comporte un quatrième volet, dit d'arrachage, qui s'étend sur le troisième volet depuis la ligne de pliage au-delà de la seconde extrémité du support.

L'unique objet de ce brevet est de proposer un conditionnement particulier du pansement dans un emballage individuel qui facilite l'application du pansement, sans offrir aucune garantie de stérilité de cette application. Pour cela, les deux volets d'arrachage des deux languettes sont scellés dans les extrémités opposées de l'emballage, qui forment deux zones de traction. Cet emballage comporte également, sur un bord longitudinal, une entaille qui est alignée avec la ligne de pliage des deux languettes, ainsi qu'une zone de préhension, disposée en regard de la deuxième languette et du support du pansement.

Pour appliquer le pansement, l'utilisateur doit maintenir l'emballage dans sa zone de préhension puis tirer sur sa première zone de traction, liée au volet d'arrachage de la première languette. L'emballage se déchire en deux parties le long de la ligne de pliage des languettes. La première languette, qui recouvre entièrement le tampon, est retirée en premier de la face adhésive du support. La deuxième languette reste en place sur le support. Dès lors, il y a un risque très élevé que l'air environnant ou les doigts de l'utilisateur viennent contaminer, avec des germes microbiens, le tampon et la plus grande partie de la face adhésive du support qui sont découverts. Lorsque cette partie contaminée est appliquée sur la zone affectée de la peau, les germes qu'elle contient peuvent provoquer des infections graves.

Un autre pansement adhésif d'un type similaire est décrit dans la demande de brevet anglais publiée sous le numéro GB-A-2 224 445. Ce pansement comprend un support pourvu d'un revêtement adhésif qui est protégé par deux languettes détachables. La première languette est repliée sur elle-même en V. Elle définit un premier volet, appliqué sur une petite partie du revêtement adhésif vers l'intérieur du support depuis une première extrémité, et un second volet d'arrachage de la première languette s'étendant vers l'extérieur du support au-delà de sa première extrémité. La deuxième languette est approximativement plane. Elle définit un troisième volet, appliqué sur une grande partie du revêtement adhésif vers l'intérieur du support depuis sa seconde extrémité, et un quatrième volet d'arrachage de la deuxième languette, qui recouvre la première languette et s'étend au-delà de la première extrémité du support.

Ce pansement adhésif ne comporte pas de tampon stérile disposé sur le revêtement adhésif et destiné à être appliqué sur la zone affectée de l'épiderme. Le seul objet de cette demande de brevet est de proposer un pansement adhésif facile à appliquer, sans offrir aucune garantie de stérilité de cette application. D'ailleurs, la deuxième languette, dont le troisième volet recouvre la plus grande partie du revêtement adhésif du support, doit être retirée en premier. De ce fait, il y a un risque très élevé de contaminer fortement, par l'air environnant et les doigts de l'utilisateur, la plus grande partie du revêtement adhésif avant son application sur la zone affectée de la peau, qui peut gravement être infectée à son tour.

On connaît d'autres pansements adhésifs similaires à ceux décrits précédemment, notamment par la demande internationale de brevet publiée sous le numéro WO 97 28771 et la demande de brevet européen publiée sous le numéro EP-A-0 308 122. Ces pansements ont divers inconvénients et, en particulier, ne garantissent pas une application stérile systématique et présentent des risques importants de contamination de la zone affectée de l'épiderme.

Par conséquent, les pansements adhésifs de l'art antérieur sont inadaptés à des applications critiques sur une zone affectée de l'épiderme très sensible à une contamination par des germes microbiens et devant rester stérile, pour lesquelles un risque, même minime, de contamination n'est pas admis.

### Exposé de l'invention

La présente invention se propose de pallier cet inconvénient majeur en fournissant un pansement adhésif pouvant être conditionné de façon stérile avant son utilisation et pourvu d'un film de protection qui permet de manipuler le pansement sans précaution particulière et d'appliquer facilement, même d'une seule main, et systématiquement de façon stérile la face adhésive et le tampon stérile du support sur la peau du patient.

Ce but est atteint par un pansement adhésif tel que décrit en préambule et caractérisé en ce que ladite deuxième languette détachable est agencée pour être enlevée de la face adhésive du support avant la première languette détachable, en ce que ledit second volet s'étend sur ledit premier volet au moins au-delà de la zone du tampon stérile en direction de ladite première extrémité du support, et en ce que ladite deuxième languette détachable comporte un quatrième volet constituant une amorce d'arrachage dudit troisième volet et qui s'étend sur ladite première languette détachable depuis ladite ligne de pliage au moins au-delà de la zone du tampon stérile en direction de ladite première extrémité du support.

Ledit quatrième volet a de préférence une longueur supérieure à celle dudit premier volet et s'étend au-delà de ladite première extrémité du support.

Ledit second volet a de préférence une longueur supérieure à celle dudit premier volet et s'étend au-delà de ladite première extrémité du support.

Selon une forme de réalisation avantageuse, ledit second volet a une longueur supérieure à celle dudit quatrième volet et s'étend au-delà dudit quatrième volet.

### Description sommaire des dessins

La présente invention et ses avantages apparaîtront mieux dans la description suivante d'un mode de réalisation préféré de l'invention, en référence aux dessins annexés, dans lesquels:
- la figure 1 est une vue latérale en coupe du pansement adhésif de l'invention,
- la figure 2 est une vue de dessous du pansement adhésif de la figure 1, et
- les figures 3a à 3g représentent les séquences de l'application du pansement adhésif des figures 1 et 2.

### Meilleure manière de réaliser l'invention

Le pansement adhésif 10 comprend un support 11, par exemple de forme sensiblement rectangulaire, pourvu d'une face adhésive 12 et d'un tampon stérile 13 disposé sur cette face adhésive 12, par exemple dans une zone centrale de celle-ci. Le pansement 10 comprend également un film de protection, objet de l'invention, qui est formé de deux languettes détachables 14 et 15.

La première languette 14 est repliée sur elle-même selon une ligne de pliage 16 sensiblement perpendiculaire à l'axe longitudinal du support 11 et en dehors de la zone du tampon stérile 13. Elle comporte un premier volet 17 appliqué sur la face adhésive 12 du support 11. Ce volet 17 s'étend depuis une première extrémité du support 11 vers l'intérieur de celui-ci jusqu'à la ligne de pliage 16, de manière à recouvrir entièrement le tampon stérile 13. La première languette 14 comporte également un second volet 18 constituant une amorce d'arrachage du premier volet 17. Ce second volet 18 s'étend sur le premier volet 17, au-delà de la zone du tampon stérile 13, depuis la ligne de pliage 16 en direction de la première extrémité du support 11. Il a une longueur supérieure à celle du premier volet 17 et s'étend au-delà de cette première extrémité du support 11.

La deuxième languette détachable 15 présente une forme approximativement plane sans zone de pliage. Elle comporte un troisième volet 19 appliqué sur la face adhésive 12 du support 11. Ce volet 19 s'étend, en dehors de la zone du tampon stérile 13, depuis une seconde extrémité du support 11, opposée à sa première extrémité, vers l'intérieur du support 11 jusqu'à la ligne de pliage 16 de la première languette 14. La deuxième languette 15 comporte un quatrième volet 20 constituant une amorce d'arrachage du troisième volet 19. Ce quatrième volet 20 s'étend sur la première languette 14, au-delà de la zone du tampon stérile 13, depuis la ligne de pliage 16 en direction de la première extrémité du support 11. Il a une longueur supérieure à celle du premier volet 17 et s'étend au-delà de la première extrémité du support 11. Ce quatrième volet 20 de la deuxième languette 15 est avantageusement plus court que le second volet 18 de la première languette 14 de sorte que ce second volet 18 s'étend au-delà du quatrième volet 20.

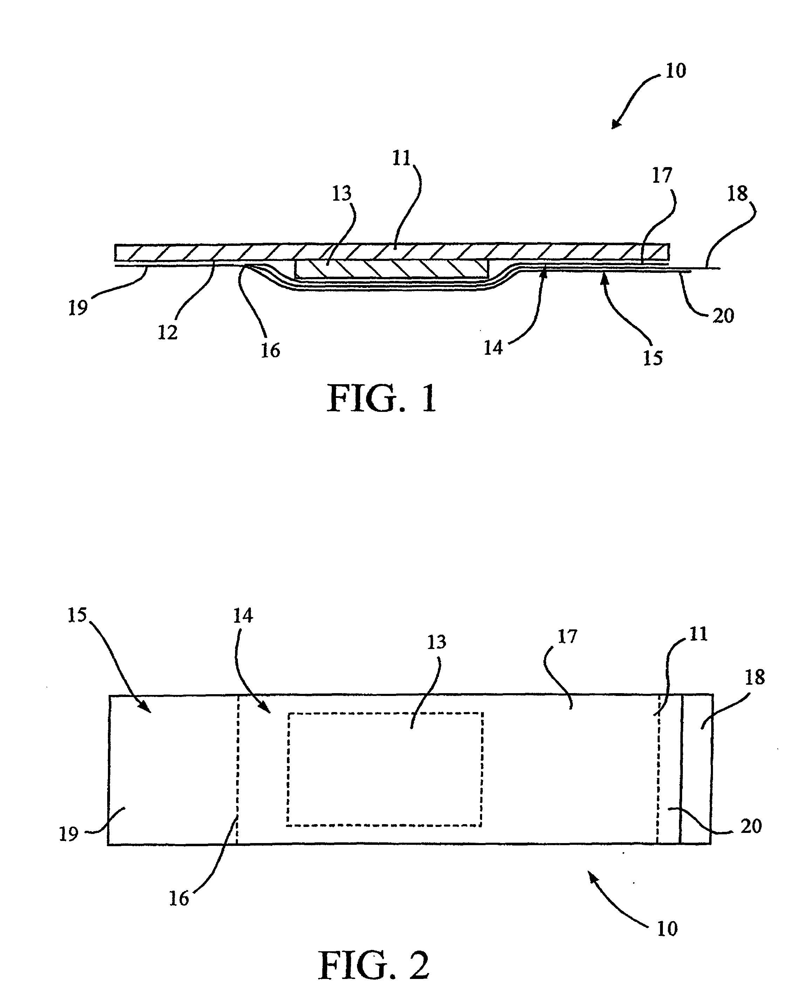

Avant son utilisation, le pansement adhésif 10 est conditionné de façon stérile dans un emballage individuel fermé. Cet emballage forme une barrière absolue pour les germes et peut aussi être étanche aux liquides. Il comporte par exemple des moyens pour faciliter son ouverture, tels qu'un prépliage ou une entaille, de sorte qu'un utilisateur peut l'ouvrir facilement, par exemple en le déchirant, sans risquer d'endommager le pansement qu'il contient. Le pansement 10 est placé dans cet emballage dans la configuration initiale décrite précédemment et illustrée par les figures 1 et 2, dans laquelle les volets 17, 18 de la première languette 14 et le quatrième volet 20 de la deuxième languette 15 sont superposés, par-dessus la zone du tampon stérile 13.

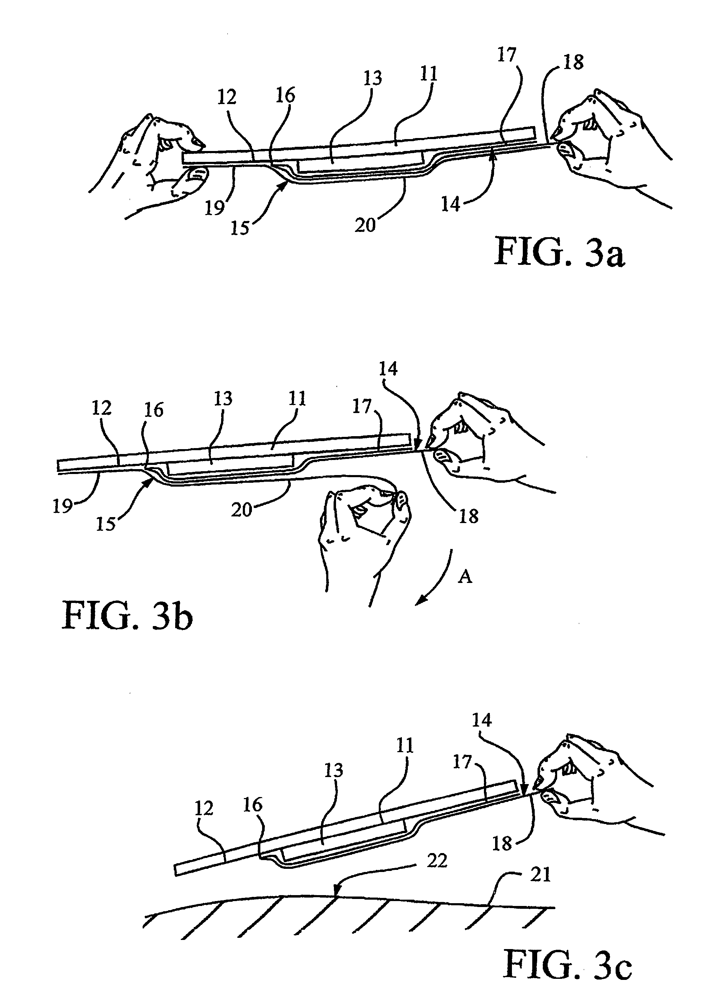

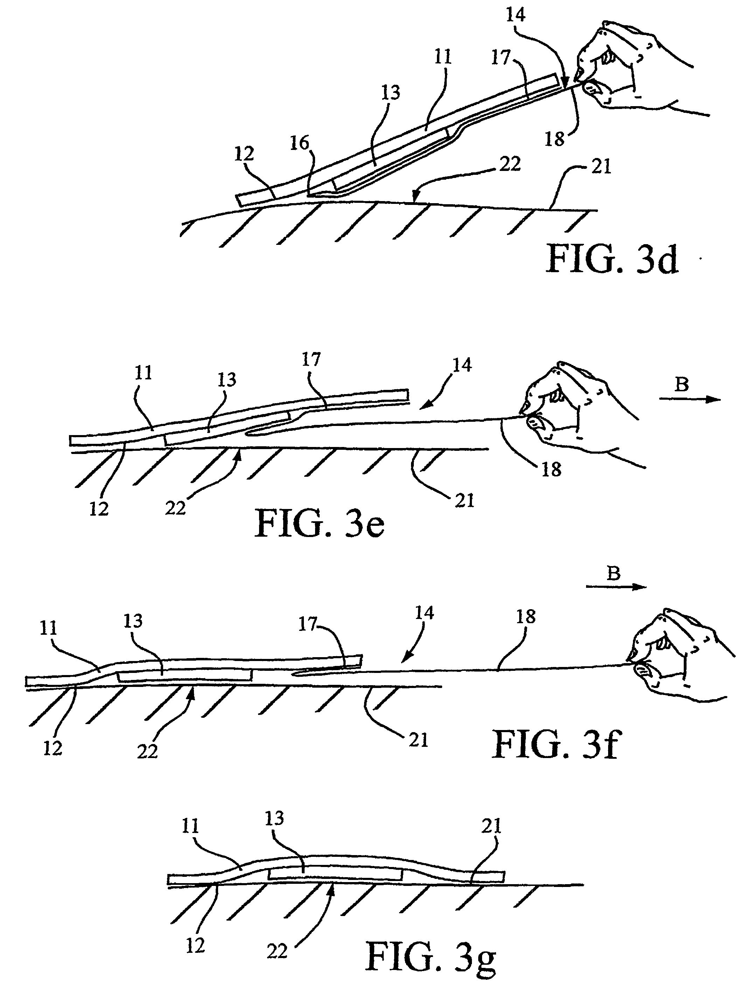

Nous allons décrire maintenant l'application du pansement adhésif 10 en référence aux figures 3a à 3g qui représentent les différentes séquences de l'application.

Tout d'abord, l'utilisateur déchire l'emballage et saisit d'une main le pansement adhésif 10 qu'il contient. La deuxième languette 15 recouvre la face adhésive 12 sur une petite partie du support 11, définie entre la seconde extrémité du support 11 et la ligne de pliage 16, ainsi que la première languette 14 sur une grande partie du support 11, définie entre la ligne de pliage 16 et la première extrémité du support 11, qui contient le tampon stérile 13. La deuxième languette 15 protègent donc efficacement ces deux parties de toute contamination par l'air environnant et par les doigts de l'utilisateur. Seule la face inférieure ou extérieure de la deuxième languette 15 peut être contaminée, mais ceci ne constitue aucun risque de contamination ultérieure pour le patient lors de l'application du pansement adhésif 10, comme cela sera expliqué ci-dessous. L'utilisateur peut donc manipuler le pansement sans aucune précaution particulière.

On se réfère maintenant à la figure 3a. Tout d'abord, l'utilisateur serre d'une main la seconde extrémité du support 11 avec le troisième volet 19 de la deuxième languette 15, puis de l'autre main vient saisir le second volet 18 d'arrachage de la première languette 14, ce qui est relativement facile car ce volet 18 s'étend au-delà du premier volet 17 et au-delà du quatrième volet 20. Ensuite, en référence à la figure 3b, il saisit avec sa première main le quatrième volet 20 d'arrachage de la deuxième languette 15, qui s'étend au-delà de la première extrémité du support 11. A cet instant, le second volet 18, du côté correspondant au quatrième volet 20, n'a jamais été en contact avec les doigts de l'utilisateur et est donc toujours stérile dans la grande partie du support 11, à droite de la ligne de pliage 16 et contenant le tampon stérile 13. L'utilisateur tire ensuite le quatrième volet 20 vers la seconde extrémité du support 11, dans le sens indiqué par la flèche A, pour décoller le troisième volet 19 de la petite partie de la face adhésive 12 du support 11, à gauche de la ligne de pliage 16, voir la figure 3c.

Cette partie de la face adhésive 12, qui est toujours stérile, peut être appliquée sur la peau 21 du patient à l'endroit approprié, c'est-à-dire à côté de la zone affectée 22 de l'épiderme devant être recouverte à la fin de l'application par le tampon stérile 13, au fur et à mesure que le troisième volet 19 se détache de la face adhésive 12 et la découvre. Ceci permet de limiter son exposition à l'air environnant.

Il est à noter que le troisième volet 19 peut être détaché entièrement de la petite partie de la face adhésive 12, comme représenté sur la figure 3c, avant que celle-ci soit collée sur la peau 21 du patient, ce qui permet de terminer l'application du pansement adhésif 10 d'une seule main. Dans ce cas, la petite partie découverte de la face adhésive 12 n'est que très légèrement et pendant peu de temps en contact avec l'air environnant et reste quasiment stérile. Toutefois, une contamination minime de la face adhésive 12 en dehors de la zone du tampon stérile 13 ne présente aucun risque de contamination pour la zone affectée 22 adjacente de l'épiderme, qui sera recouverte à la fin de l'application par le tampon stérile 13.

En référence maintenant à la figure 3d, la petite partie de la face adhésive 12, à gauche de la ligne de pliage 16, est désormais collée sur la peau 21 du patient. La face inférieure du second volet 18, qui était jusqu'à présent protégée par le quatrième volet 20 de la deuxième languette 15, est maintenant en regard de la peau 21 du patient et de la zone affectée 22 de l'épiderme. Cependant, cette face du second volet 18 n'a jamais été en contact avec les doigts de l'utilisateur, en particulier dans la zone du tampon stérile 13, et peut donc toucher la zone affectée 22 de l'épiderme sans aucun risque de contamination. Il est également important de souligner que la grande partie de la face adhésive 12 du support 11, à droite de la ligne de pliage 16, et le tampon stérile 13 sont encore totalement recouverts par le premier volet 17 de la première languette détachable 14 et sont toujours parfaitement stériles.

Finalement et en référence aux figures 3e à 3g, l'utilisateur tire le second volet 18 d'arrachage de la première languette détachable 14 vers l'extérieur du support 11, dans le sens indiqué par la flèche B, pour décoller le premier volet 17 de la grande partie de la face adhésive 12 du support 11, à droite de ta ligne de pliage 16. La face adhésive 12 découverte et stérile du support 11 se colle au fur et à mesure sur la peau 21 du patient, le tampon stérile 13 venant s'appliquer de manière parfaitement stérile sur la zone affectée 22 de l'épiderme.

Le film de protection du pansement adhésif 10 de l'invention a de nombreux avantages. La deuxième languette détachable 15 du film de protection protège efficacement le pansement 10 quand il est sorti de son emballage et permet de le manipuler sans précaution particulière et sans aucun risque de contamination ultérieure pour le patient. Les volets d'arrachage 18, 20 des languettes détachables 14, 15 permettent d'appliquer facilement le pansement 10 sur la peau, même d'une seule main, et ceci systématiquement de façon stérile. En effet, les faces des volets 17 à 20 pouvant être en contact avec la zone affectée 22 de l'épiderme lors de l'application du support 11 n'ont jamais été en contact avec les doigts de l'utilisateur. Elles sont donc toujours stériles et ne risquent pas de la contaminer. De plus, la face adhésive 12 et le tampon stérile 13 du support 11 sont appliqués rapidement sur la peau 21 du patient, dès qu'ils sont découverts, ce qui élimine également les risques d'une contamination par l'air environnant.

Le pansement adhésif 10 de la présente invention est particulièrement bien adapté à des applications nécessitant une stérilité optimale et pour lesquelles le moindre risque de contamination de la zone affectée de l'épiderme peut avoir des conséquences graves, voire fatales, et doit être supprimé. Des exemples d'applications critiques sont celles réalisées dans un bloc opératoire, notamment pour le recouvrement de plaie de laparotomie, de thoracotomie, de brûlure ou similaire.

Une application particulièrement critique d'un pansement adhésif concerne le domaine de la dialyse. Un patient atteint de problèmes rénaux chroniques est tenu de suivre régulièrement, parfois quotidiennement, un traitement de dialyse du sang en milieu hospitalier ou à domicile. A chaque séance, une artère et une veine du circuit sanguin du patient doivent être connectées à une machine de dialyse pour épurer le sang du patient malade. Afin de faciliter ce traitement et soulager le patient, ce dernier porte en permanence une fistule artérioveineuse implantée, lors d'une intervention chirurgicale, sous l'épiderme de l'avant-bras, et connectée à chaque séance à la machine de dialyse. Cette fistule forme un abord vasculaire à demeure sur le patient, qui doit toujours être recouvert d'un pansement adhésif stérile pour deux raisons. Ce pansement sert d'une part à maintenir l'abord vasculaire, qui peut se détacher de l'avant-bras, pour éviter sa perte et une nouvelle intervention chirurgicale pour le recréer, et d'autre part et principalement à le protéger d'une contamination par des germes microbiens afin d'éviter une septicémie latente chez un patient déjà malade et fragile. Pour garantir une protection et une stérilité durables, le pansement adhésif appliqué sur l'abord vasculaire du bras doit être remplacé régulièrement, au moins après chaque séance, et souvent par le patient lui-même, ceci forcément d'une seule main. Par conséquent, le pansement adhésif utilisé doit permettre systématiquement une application parfaitement stérile, et ceci même d'une seule main sans aucun risque de contamination particulier supplémentaire. Le pansement adhésif 10 de l'invention répond à ces critères sévères d'application.

Bien entendu, le pansement adhésif 10 de l'invention convient à d'autres applications moins sévères en ce qui concerne la stérilité. Le pansement adhésif peut être du type pansement occlusif, le support 11 et le tampon stérile 13 étant alors perméables aux gaz, mais imperméables aux liquides et aux bactéries. Le support 11 peut être transparent, par exemple formé en polyuréthanne, afin de visualiser la surface de peau 21 recouverte. La face adhésive 12 peut être également hypoallergénique. Le pansement adhésif 10 peut être hydroactif, le tampon stérile 13 contenant alors de l'eau additionnée d'autres agents permettant sa décomposition, par exemple pour favoriser la guérison d'une plaie. Le pansement adhésif 10 peut avoir n'importe quelles dimensions, petites ou grandes, et n'importe quelles formes, rectangulaires, ovales, ou circulaires par exemple.

Le pansement adhésif peut être fabriqué de façon simple et économique sur des machines conventionnelles. Une seule languette détachable 14 est repliée sur elle-même, l'autre languette détachable 15 n'ayant pas de zone de pliage. Comme la deuxième languette 15 recouvre le support sur toute sa longueur, la tolérance relative à la position de la ligne de pliage 16 de la première languette 14 sur la face adhésive 12 peut être large. Il suffit simplement qu'elle soit disposée en dehors de la zone du tampon stérile 13. Le pliage de la languette détachable 14 se fait toujours en dehors de la zone du tampon stérile 13, ce dernier conservant toute son efficacité. En outre, le pansement adhésif 10 peut être conditionné de façon stérile dans un emballage individuel quelconque, la deuxième languette détachable 15 protégeant efficacement le pansement 10 quand il est sorti de son emballage.

La présente invention n'est pas limitée au mode de réalisation décrit et illustré, mais s'étend à toute modification et variante évidente pour l'homme du métier. Par exemple, le support 11 peut être recouvert, sur sa face supérieure non adhésive, d'une couche de renforcement détachable qui est enlevée après l'application du support sur la peau. Cela permet d'une part, d'éviter le froissement du support lors de l'application si celui-ci est trop souple et, d'autre part, d'éviter le contact des doigts sur la face supérieure du support pour améliorer la stérilité de l'application.

Pour garantir la stérilité de l'application du tampon 13 sur la zone affectée de l'épiderme, il est seulement essentiel que le quatrième volet 20 de la deuxième languette détachable 15 s'étendent sur le second volet 18 de la première languette détachable 14 et que ces deux volets d'arrachage 18, 20 s'étendent depuis la ligne de pliage 16, au moins au-delà de la zone du tampon stérile 13 en direction de la première extrémité du support 11. Le fait que ces deux volets 18, 20 soient plus longs que le premier volet 17 et s'étendent au-delà de la première extrémité du support 11 permet d'une part de faciliter la manipulation du pansement 10 lors de son application et d'autre part de garantir, outre la stérilité de l'application du tampon 13 sur la zone affectée de l'épiderme, la stérilité de l'application de la face adhésive 12 du support 11 sur la peau du patient entourant la zone affectée 22 recouverte par le tampon stérile 13. D'ailleurs, le quatrième volet 20 de la deuxième languette détachable 15 peut avoir une longueur supérieure à celle du second volet 18 de la première languette détachable 14 et s'étendre au-delà de la première extrémité du support 11 et du second volet 18.

## Revendications

1. Pansement adhésif, en particulier pour une application sur une zone affectée critique de l'épiderme, très sensible à une contamination par des germes microbiens et devant rester stérile, notamment une zone comportant un site d'implantation d'une fistule artérioveineuse formant un abord vasculaire d'un patient en hémodialyse, ledit pansement adhésif comprenant un support ayant une face adhésive, un tampon stérile disposé sur ladite face adhésive du support et un film de protection recouvrant la face adhésive et le tampon stérile, ledit film de protection étant formé d'une première et d'une deuxième languette détachable, ladite première languette détachable étant repliée sur elle-même selon une ligne de pliage, en dehors de la zone du tampon stérile, et comportant un premier volet appliqué sur la face adhésive du support et qui s'étend au moins d'une première extrémité dudit support vers l'intérieur de celui-ci jusqu'à ladite ligne de pliage de manière à recouvrir entièrement le tampon stérile, et un second volet constituant une amorce d'arrachage dudit premier volet et qui s'étend sur ledit premier volet depuis ladite ligne de pliage en direction de ladite première extrémité du support, ladite deuxième languette détachable comportant un troisième volet appliqué sur la face adhésive du support et qui s'étend, en dehors de la zone du tampon stérile, au moins d'une seconde extrémité dudit support, opposée à ladite première extrémité, vers l'intérieur dudit support jusqu'à ladite ligne de pliage de la première languette détachable, **caractérisé en ce que** ladite deuxième languette détachable (15) est agencée pour être enlevée de la face adhésive (12) du support (11) avant la première languette détachable (14), **en ce que** ledit second volet (18) s'étend sur ledit premier volet (17) au moins au-delà de la zone du tampon stérile (13) en direction de ladite première extrémité du support (11), et **en ce que** ladite deuxième languette détachable (15) comporte un quatrième volet (20) constituant une amorce d'arrachage dudit troisième volet (19) et qui s'étend sur ladite première languette détachable (14) depuis ladite ligne de pliage (16) au moins au-delà de la zone du tampon stérile (13) en direction de ladite première extrémité du support (11).

2. Pansement adhésif selon la revendication 1, **caractérisé en ce que** ledit quatrième volet (20) a une longueur supérieure à celle dudit premier volet (17) et s'étend au-delà de ladite première extrémité du support (11).

3. Pansement adhésif selon la revendication 1, **caractérisé en ce que** ledit second volet (18) a une longueur supérieure à celle dudit premier volet (17) et s'étend au-delà de ladite première extrémité du support (11).

4. Pansement adhésif selon les revendications 2 et 3, **caractérisé en ce que** ledit second volet (18) a une longueur supérieure à celle dudit quatrième volet (20) et s'étend au-delà dudit quatrième volet (20).

## Claims

1. An adhesive bandage, in particular, for application on an acutely affected area of the epidermis which is very sensitive to contamination by bacterial germs and must remain sterile, notably, an area comprising a site for the implementation of an arteriovenous fistula giving vascular access to a patient on hemodialysis, the said adhesive bandage comprising a support with an adhesive side, a sterile pad situated on the said adhesive side of the support and a protective film covering the adhesive side and the sterile pad, the said protective film being made up of a first and a second detachable tab, the said first detachable tab being folded back on itself along a folding line, outside the sterile pad area and comprising a first flap applied on the adhesive side of the support and which extends at least from a first extremity of the said support towards its interior until the said folding line in such a way as to cover entirely the sterile pad, and a second flap consisting of a beginning which tears off from the said first flap and which extends over the said first flap from the said folding line in the direction of the said first extremity of the support, the said second detachable tab comprising a third flap applied on the adhesive side of the support, and which extends, outside the area of the sterile pad, at least from a second extremity of the said support, opposite the said first extremity, towards the interior of the said support until the said folding line of the first detachable tab, **characterised in that** the said second detachable tab (15) is arranged to be lifted from the adhesive side (12) of the support (11) before the first detachable tab (14), **in that** the said second flap (18) extends over the said first flap (17) at least beyond the area of the sterile pad (13) in the direction of the said first extremity of the support (11), and **in that** the said second detachable tab (15) comprises a fourth flap (20) consisting of a beginning which tears off from the said third flap (19) and which extends over the said first detachable tab (14) from the said folding line (16) at least beyond the area of the sterile pad (13) in the direction of the said first extremity of the support (11).

2. An adhesive bandage according to claim 1, **characterised in that** the said fourth flap (20) has a length greater than that of the said first flap (17) and extends beyond the said first extremity of the support (11).

3. An adhesive bandage according to claim 1, **characterised in that** the said second flap (18) has a length greater than that of the said first flap (17) and extends beyond the said first extremity of the support (11).

4. An adhesive bandage according to claims 2 and 3, **characterised in that** the said second flap (18) has a length greater than that of the said fourth flap (20) and extends beyond the said fourth flap (20).

## Patentansprüche

1. Heftpflaster, insbesondere zur Anwendung in einer kritischen betroffenen Zone der Epidermis, die empfindlich für eine Ansteckung durch mikrobielle krankheitserregende Keime ist und steril bleiben muss, insbesondere in einer Zone mit implantierter arteriovenöser Fistel, die einen Gefäßzugang für einen Hämodialysepatienten bildet, wobei das Heftpflaster einen Träger mit einer klebenden Seite, einen auf der klebenden Seite des Trägers angeordneten sterilen Tupfer und eine die klebende Seite und den sterilen Tupfer abdeckende Schutzfolie aufweist, wobei die Schutzfolie aus einer ersten und einer zweiten lösbaren Lasche besteht, wobei die erste lösbare Lasche entsprechend einer Falzlinie außerhalb der Zone des sterilen Tupfers auf sich selbst gefaltet ist und eine auf die klebende Seite des Trägers aufgebrachte erste Klappe, die sich mindestens von einem ersten äußersten Ende des Trägers in Richtung des Inneren desselben bis zu der Falzlinie so erstreckt, dass sie den sterilen Tupfer vollständig abdeckt, und eine zweite Klappe aufweist, die eine Abreißlasche der ersten Klappe darstellt und sich ab der Falzlinie über die erste Klappe in Richtung des ersten äußersten Endes des Trägers erstreckt, wobei die zweite lösbare Lasche eine auf die klebende Seite des Trägers aufgebrachte dritte Klappe aufweist, die sich außerhalb der Zone des sterilen Tupfers mindestens von einem dem ersten äußersten Ende gegenüberliegenden, zweiten äußersten Ende des Trägers in Richtung des Inneren des Trägers bis zu der Falzlinie der ersten lösbaren Lasche erstreckt,
**dadurch gekennzeichnet, dass**
die zweite lösbare Lasche (15) so angeordnet ist, dass sie vor der ersten lösbaren Lasche (14) von der klebenden Seite (12) des Trägers (11) abgezogen werden kann, wobei sich die zweite Klappe (18) mindestens über die Zone des sterilen Tupfers (13) hinaus in Richtung des ersten äußersten Endes des Trägers (11) erstreckt, und wobei die zweite lösbare Lasche (15) eine vierte Klappe (20) aufweist, die eine Abreißlasche der dritten Klappe (19) darstellt und sich ab der Falzlinie (16) über die erste lösbare Lasche (14) mindestens über die Zone des sterilen Tupfers (13) hinaus in Richtung des ersten äußersten Endes des Trägers (11) erstreckt.

2. Heftpflaster nach Anspruch 1,
**dadurch gekennzeichnet, dass**
die vierte Klappe (20) eine Länge aufweist, die größer ist als diejenige der ersten Klappe (17), und sich über das erste äußerste Ende des Trägers (11) hinaus erstreckt.

3. Heftpflaster nach Anspruch 1,
**dadurch gekennzeichnet, dass**
die zweite Klappe (18) eine Länge aufweist, die größer ist als diejenige der ersten Klappe (17), und sich über das erste äußerste Ende des Trägers (11) hinaus erstreckt.

4. Heftpflaster nach den Ansprüchen 2 und 3,
**dadurch gekennzeichnet, dass**
die zweite Klappe (18) eine Länge aufweist, die größer ist als diejenige der vierten Klappe (20), und sich über die vierte Klappe (20) hinaus erstreckt.
